# EUROPEAN PATENT SPECIFICATION

(11) **EP 2 426 138 B1**
(45) Date of publication and mention of the grant of the patent: **01.07.2015**
(21) Application number: 10769609.8
(22) Date of filing: 13.04.2010
(51) Int. Cl.: A23L 2/52, A23L 1/305, C07K 14/78, C07K 1/12, A23J 3/34, C12P 21/06, C07K 5/083, C07K 5/078

(54) **Collagen peptide composition having good blood transfer properties, and food and drink containing same**
Kollagenpeptidzusammensetzung mit guten Blutübertragungseigenschaften sowie Nahrungsmittel und Getränk damit
Composition peptidique de collagène ayant de bonnes propriétés de transfert sanguin, et aliments et boissons la contenant

(30) Priority: 28.04.2009 JP 2009109171
(43) Date of publication of application: 07.03.2012
(73) Proprietor: Meiji Co., Ltd., Tokyo 136-8908 (JP)
(72) Inventor: OHARA, Hiroki, Koto-ku Tokyo 136-8908 (JP); MATSUMOTO, Hitoshi, Koto-ku Tokyo 136-8908 (JP); HATA, Masataka, Minamikawachi-gun Osaka 583-0996 (JP); TERAUCHI, Koichi, Yao-shi Osaka 581-0034 (JP); NISHIZAWA, Hidetoshi, Itami-shi Hyogo 664-0001 (JP)
(74) Representative: Gillard, Richard Edward
(86) International application number: PCT/JP2010/056596
(87) International publication number: WO 2010/125910

(56) References cited:
- WO-A1-01/84943
- WO-A1-2008/059927
- JP-A- 2002 051 734
- JP-A- 2005 314 265
- JP-A- 2006 151 847
- JP-A- 2008 220 208

## Description

### Technical Field

The present invention relates to a collagen peptide composition having good ability to enter the blood and a food or beverage containing the collagen peptide composition.

### Background Art

Collagen is one of proteins that constitute dermis, ligament, tendon, bone, cartilage, and the like, and it is a major ingredient of the extracellular matrix of multicellular animals. Collagen is present everywhere throughout skin, blood vessels, viscera, bone tissues, and the like, and it accounts for about 30% of body-forming proteins. In the skin, 70% of dermis is made up of collagen. Fascia wrapping individual muscles is also made up of collagen.

In recent years, collagen has been found to exert various physiological and pharmacological effects such as bone strengthening effects that lead to prevention and/or improvement of osteoporosis (Patent Document 1), effects for accelerating metabolism in living tissue, which ameliorate the declining functions of living tissues with aging (Patent Document 2), skin metabolism accelerating effects, skin activating effects (Patent Document 3), and antiaging effects for skin in order to prevent and/or improve wrinkles (Patent Document 4). Collagen is broadly used as a raw material for cosmetics and food products or as a biologically functional material for pharmacological products.

Gelatin prepared by heat denaturation of collagen has a high molecular weight. Hence, gelatin that is actually absorbed through digestion is a collagen peptide prepared by hydrolysis of gelatin to have a lower molecular weight. It is known that when a collagen peptide is orally ingested, it is absorbed through digestion in the form of an amino acid, dipeptide, tripeptide, or the like. However, the sufficient *in vivo* exertion of the above physiological and pharmacological effects of collagen peptides require the high intake thereof. Also, it has been reported that the effective daily intake of a collagen peptide required for sufficient improvement of dry human skin when orally ingested by a human ranges from 5 g to 10 g for a fish scale collagen peptide and is 10 g or more for a pig skin collagen peptide (Non-patent Document 1). However, long-term high intake of only a specific protein in addition to general meals is difficult and is unfavorable from a nutritional standpoint.

Hence, the active component of collagen peptides has been studied for collagen peptides to effectively exert their effects even with a low intake thereof. For example, Taniguchi et al., have reported that as a result of comparison and examination of collagen hydrolysates (a peptide mixture or peptides) and an amino acid mixture having the same proportion as the collagen hydrolysates in terms of effects of accelerating skin collagen synthesis in rats, the amino acid mixture has been observed to exert no effects and only the collagen hydrolysates have been observed to exert effects (Non-Patent Document 2). Collagen hydrolysates are known to be digested into amino acids, dipeptides, or tripeptides during digestion and absorption thereof. Taniguchi's report has suggested that effects are exerted not by amino acids, but by dipeptides or tripeptides. It has also been reported that Pro-Hyp, that is, one of collagen peptide-derived hydroxyproline-containing dipeptides, activates the cell growth when caused to act on skin fibroblasts and accelerates the transcription of hyaluronic acid synthase so as to accelerate hyaluronic acid production (Non-patent Document 3). It has also been reported that collagen hydrolysates containing tripeptides having the amino acid sequence of Gly-X-Y have collagen synthesis-accelerating activity (Patent Documents 5 and 6).

As described above, it has been revealed that dipeptides or tripeptides having specific compositions serve as the active component of a collagen hydrolysate (collagen peptide). However, there are few studies concerning the composition of a collagen peptide for efficiently causing such a dipeptide or a tripeptide serving as the active component to enter the blood. For example, Patent Document 7 discloses that a collagen peptide composition having a specific molecular weight distribution exerts excellent usability and skin permeability when mixed with skin cosmetics or pharmacological products. However, this document does not disclose any examination concerning the evaluation of the ability to enter the blood in the case of oral ingestion. In contrast, the present inventors have reported that a collagen peptide composition having a specific molecular weight distribution and the ratio of glycine to total of N-terminal amino acid residues of the peptides of the composition is within a specific range, has good ability to enter the blood (Patent document 8). However, high intake is required even in the case of such a collagen peptide composition.

JP 2008 220208 discloses a method for producing a low-molecular collagen. WO 01/84943 relates to acidic milk drinks composed of an acidic milk drink base together with collagen peptides obtained by degrading collagen and stabilizers. WO 2008/059927 relates to a collagen peptide composition produced by digesting a collagen or gelatin with a protease.

### Prior Art Documents

### Patent Documents

[Patent Document 1] JP Patent Publication (Kokai) No. 9-255588 A (1997)
[Patent Document 2] JP Patent Publication (Kokai) No. 7-278012 A (1995)
[Patent Document 3] JP Patent Publication (Kokai) No. 9-67262 A (1997)
[Patent Document 4] JP Patent Publication (Kokai) No. 2005-314265 A
[Patent Document 5] JP Patent Publication (Kokai) No. 2001-131084 A
[Patent Document 6] JP Patent Publication (Kokai) No. 2003-137807 A
[Patent Document 7] JP Patent Publication (Kokai) No. 2006-151847 A
[Patent Document 8] WO2008-059927

### Non-Patent Documents

[Non-PatentDocument 1] Nippon Shokuhin Kagaku Kogaku Kaishi, 2009, March, Vol. 56, No. 3, p. 144-152
[Non-Patent Document 2] Lecture Summaries of The Meeting of The Japanese Society of The Veterinary Science, 2001, September 7, Vol. 132, p. 126, PS-5014
[Non-Patent Document 3] Journal of Dermatological Science, 2007, July, Vol. 47, p. 102, 179

### Disclosure of the Invention

### Problems to be solved by the Invention

It is thought that peptides enter the blood via two pathways: a pathway mediated by a peptide transporter of the small intestine epithelium and a pathway whereby peptides pass through a tight junction. However, most peptides that pass through these pathways are dipeptides and tripeptides.

Also, a collagen peptide as a protein is digested within the gastrointestinal tract, such as in the stomach or intestine, by gastric acid or an enzyme such as protease. Hence, direct oral ingestion of dipeptides or tripeptides is unfavorable since they are digested by an enzyme or the like. Therefore, an object of the present invention is to elucidate a collagen peptide that is optimum for generation of dipeptides or tripeptides serving as the active component of collagen and is effective for causing dipeptides or tripeptides to enter the blood, so that required intake may be reduced.

### Means for solving the problems

As a result of intensive studies concerning a collagen peptide that is effective for causing active dipeptides or active tripeptides to enter the blood via the gastrointestinal tract after oral ingestion of the collagen peptide, the present inventors have elucidated that the X-Hyp-Gly-Y peptide (where X denotes one arbitrary amino acid residue and Y denotes one, two or more arbitrary amino acid residues) in which hydroxyproline (Hyp) is present at the second position and glycine (Gly) is present at the third position from the N terminus is appropriate.

Based on the above findings, the present inventors have further examined a collagen peptide composition for efficient ingestion of the X-Hyp-Gly-Y peptide in which hydroxyproline (Hyp) is present at the second position and glycine (Gly) is present at the third position from the N terminus. The present inventors have elucidated that it is effective for the ratio of hydroxyproline to total of amino acid residues at the second position from the N terminus of the peptides in the composition, the ratio of glycine to total of amino acid residues at the third position from the N terminus of the same, and the average molecular weight of the composition to be within predetermined ranges. Thus, the present inventors have completed the present invention.

The present invention encompasses the following (1) to (5).
(1) A collagen peptide composition obtainable by digesting collagen or gelatin derived from fish scale with protease for 0.5 to 4 hours at 30 to 80°C, wherein the gelatinized collagen or gelatin is prepared by acid treatment of collagen raw materials; and wherein
   (a) the ratio of hydroxyproline to total of amino acid residues at the second position from the N terminus of the peptides in the composition is 2 mol% or more and 20 mol% or less and the ratio of glycine to total of amino acid residues at the third position from the N terminus of the same is 20 mol% or more and 50 mol% or less; and
   (b) the average molecular weight is 1000 or more and 1500 or less;
   (c) wherein the protease is papain alone or an enzyme mixture of papain and one, two or more types of neutral protease from the genus Aspergillus or the genus Bacillus, or neutral protease from the genus Rhizopus.; and
   (d) the papain has specific activity ranging from 400 U/g to 50000 U/g.
(2) A food or beverage, containing the collagen peptide composition according to (1).

This application claims priority of Japanese patent application No. 2009-109171 filed on April 28, 2009.

### Effect of the Invention

According to the present invention, a collagen peptide composition is provided that is capable of causing dipeptides or tripeptides serving as the active component to enter the blood more efficiently than conventional collagen peptides. The ability to enter the blood is 1.7 to 1.9 times that of conventional fish scale collagen peptides. Thus the effective intake thereof can be reduced to about a half that of conventional products. Accordingly, a food or beverage is mixed with the collagen peptide composition of the present invention and is then orally ingested, so that the physiological and pharmacological effects can be exerted efficiently in an amount smaller than that of conventional products.

### Brief Description of the Drawings

Fig. 1 shows the molecular weight distribution of collagen peptide composition 1 of the present invention.
Fig. 2 shows the molecular weight distribution of collagen peptide composition 2 of the present invention.
Fig. 3 shows the molecular weight distribution of collagen peptide composition 3 of the present invention.
Fig. 4 shows the molecular weight distribution of collagen peptide composition 4 of the present invention.
Fig. 5 shows the molecular weight distribution of collagen peptide composition 5 (reference example).
Fig. 6 shows the molecular weight distribution of collagen peptide composition 6 (reference example).

### Best Mode for Carrying Out the Invention

The present invention is described in detail as follows.

### 1. Collagen peptide composition

The collagen peptide composition of the present invention is obtained by digesting gelatinized collagen or gelatin with protease, wherein:
(a) the ratio of hydroxyproline to total of amino acid residues at the second position from the N terminus of the peptides in the composition is 2 mol% or more and 20 mol% or less and the ratio of glycine to total of amino acid residues at the third position from the N terminus of the same is 20 mol% or more and 50 mol% or less; and
(b) the average molecular weight is 1000 or more and 1500 or less.

Here, "the ratio of hydroxyproline to total of amino acid residues at the second position from the N terminus" refers to a value obtained by analyzing the amino acid residue at the second position from the N terminus of each peptide in the collagen peptide composition and then expressing the ratio of the number of moles of hydroxyproline to the total number of moles of amino acid residues at the second position from the N terminus detected by the analysis in terms of molar percentage (mol%). Also, "the ratio of glycine to total of amino acid residues at the third position from the N terminus" refers to a value obtained by analyzing the amino acid residue at the third position from the N terminus of each peptide in the collagen peptide composition and then expressing the ratio of the number of moles of glycine to the total number of moles of amino acid residues at the third position from the N terminus detected by the analysis in terms of molar percentage (mol%). The above analysis of amino acids may be performed using an amino acid sequence analyzer used for carrying out the Edman method in an automated manner.

The ratio of hydroxyproline to total of amino acid residues at the second position from the N terminus of the peptides in the above composition is 2 mol% or more and 20 mol% or less, and is preferably 2 mol% or more and 10 mol% or less. Also, the ratio of glycine to total of amino acid residues at the third position from the N terminus of the peptides in the above composition is 20 mol% or more and 50 mol% or less, and is preferably 30 mol% or more and 45 mol% or less.

The average molecular weight of the collagen peptide composition of the present invention is 1000 or more and 1500 or less. The collagen peptide composition having a molecular weight of higher than 2000 takes much time for digestion. Moreover, the time for exposure thereof to an exo-type enzyme within the gastrointestinal tract is prolonged, the amount of amino acids generated is increased, and thus the physiological activity of the collagen peptide disappears. Also, when the collagen peptide composition has a molecular weight of less than 500, an endo-type enzyme acts as an exo-type enzyme on the second residue from the N terminus, the collagen peptide composition is digested to amino acids, the amount of amino acids generated is increased, and thus the physiological activity of the collagen peptide disappears. The average molecular weight may be measured using gel filtration high performance liquid chromatography. The average molecular weight is calculated as weight average molecular weight.

The collagen peptide composition of the present invention is characterized by having high ability to enter the blood in the form of dipeptide or tripeptide (absorbability). Examples of "dipeptide" include Pro-Hyp, Ile-Hyp, Leu-Hyp, and Phe-Hyp. Examples of "tripeptide" include Pro-Hyp-Gly, Ala-Hyp-Gly, and Ser-Hyp-Gly.

The term "collagen peptide composition" (hereinafter, may also be simply referred to as "collagen peptide" in this description) to be used in the present invention refers to a mixture of peptides that are obtained by hydrolysis of collagen or gelatin.

Collagen or gelatin that is used as a raw material for the "collagen peptide composition" to be used in the present invention is derived from fishes such as shark.

Collagen is obtained from the fish scale portions. Prior to use fish scales, a step of washing (e.g., washing with water) is preferably carried out in advance for several times to remove dirt or contaminants adhered on fish scales, defatting is carried out to remove fat and oil contents, and then decalcification is carried out to remove inorganic materials such as phosphorus and calcium.

Also, when collagen is used as a raw material, collagen is preferably gelatinized once. Gelatin is prepared by heat-denaturing and solubilizing collagen. Gelatinization is carried out by pretreating a collagen raw material with acid, followed by heating and extraction. Acid treatment is carried out by immersing a collagen raw material in an inorganic acid such as hydrochloric acid or sulfuric acid for 0.5 to 48 hours and preferably for 1 to 4 hours. Moreover, the pretreated raw material is washed with water to remove excessive acid, subjected to 1st extraction with hot water at 40°C to 80°C, and then subjected to 2nd extraction and subsequent extraction with hot water at a temperature higher than that used for the 1st extraction.

### 2. Production of collagen peptide composition

A "collagen peptide composition" to be used in the present invention is produced as described below, for example. Protease treatment is carried out for collagen or gelatin obtained from collagen by the above treatment so as to digest the collagen molecules into the form of peptide. As protease to be used herein, papain alone, or, an enzyme mixture of papain and one, two or more types of another protease is used, so that the ratio of hydroxyproline to total of amino acid residues at the second position from the N terminus, the ratio of glycine to total of amino acid residues at the third position from the N terminus, and the average molecular weight of the collagen peptide composition are within the above predetermined ranges. Papain is protease that is extracted from papaya (*Carica Papaya* L) fruit milk. Purified papain having high enzyme activity is preferably used. "Purified papain" to be used in the present invention has specific activity ranging from 400 U/g to 5000 U/g and preferably ranging from 700 U/g to 1000 U/g. Specific examples of such purified papain include commercial products such as purified papain (Trade name) (Mitsubishi-Kagaku Foods Corporation) and papain 30000ES (Trade name) (Genencor).

Also, as another protease, neutral protease or alkaline protease can be used. Neutral protease is preferable and neutral protease from the genus *Aspergillus* or the genus *Bacillus,* or neutral protease from the genus *Rhizopus* is more preferable. Specific examples thereof include Neutrase (Trade name) (Novozymes Japan), protease P "Amano" 3G (Trade name) (Amano Enzyme Inc.), protease A "Amano" G (Trade name) (Amano Enzyme Inc.), protease N "Amano" G (Trade name) (Amano Enzyme Inc.), protease S "Amano" G (Trade name) (Amano Enzyme Inc.), Sumizyme FP (Trade name) (Shinnihon Chemicals), Sumizyme LP (Trade name) (Shinnihon Chemicals), Protin PC10F (Trade name) (Daiwa Fine Chemicals Co., Ltd.), Denazyme AP (Trade name) (Nagase ChemteX Corporation), and peptidase R (Trade name) (Amano Enzyme Inc.).

Enzyme treatment is carried out by adding an enzyme so that the total activity of the enzyme in 100 g of collagen or gelatin ranges from 400 U to 5000 U and preferably ranges from 400 U to 2000 U when purified papain is used, for example. Here, the term "total activity (U) of an enzyme" refers to the product of enzyme specific activity (U/g) × weight (g) of the enzyme used herein. Also, enzyme treatment is carried out at 30°C to 80°C for 0.5 to 4 hours. The unit of activity, U, of the above enzyme can be found by a measurement method (7th Edition, Japanese Standards of Food Additives, p. 378-379, 1999) using casein as a substrate. The above temperature and time for treatment are just examples and may be adequately adjusted to enable sufficient exertion of enzyme functions in order to obtain a collagen peptide composition wherein a target average molecular weight and the ratios of specific amino acid residues at the second position and at the third position from the N terminus are within the predetermined ranges.

After the above enzyme treatment, the resultant is heat-treated at 80°C to 100°C, so as to inactivate the enzyme. Excessive high-temperature treatment is undesirable since such treatment may destroy the flavor.

At the stage after completion of the above enzyme treatment, a collagen peptide composition is in a state of being dissolved or dispersed in an enzyme treatment solution. The collagen peptide composition can be purified from the enzyme solution by various generally employed purification means. Such purification means are not particularly limited. For example, improvement of color tone and flavor and removal of impurities can be very conveniently carried out by the addition of activated carbon. Impurities can also be removed by conventionally known solid-liquid separation such as filtration or centrifugation. A collagen peptide solution treated as described above is dried by a method such as spray drying or using a drum dryer, so that powderization can be carried out.

### 3. Food or beverage containing collagen peptide composition

The collagen peptide composition is **characterized in that** the collagen peptide in the form of dipeptide or tripeptide has good ability to enter the blood, so that it can be provided as a food or beverage for daily intake. Examples of the forms of the collagen peptide composition in foods or beverages include a form such that the collagen peptide composition is directly a food or beverage and a form such that the collagen peptide composition is a raw material or an intermediate product upon production of a food or beverage.

In the present invention, the term "food(s) or beverage(s)" is used to include health foods, functional foods, foods for specified health use, foods for sick or injured persons, foods for nursing care, and the like. Moreover, when such food or beverage of the present invention is used for mammals other than humans, birds, and fishes, the term can be used to include a feedstuff.

The form of a food or beverage to be mixed with the collagen peptide composition may be either a solid form or a liquid form. Specific examples of the types of foods or beverages include, but are not limited to, beverages such as soft drinks, carbonated drinks, nutritional beverages, fruit beverages, and milk beverages (including a concentrated stock solution of such a beverage and a dry powder for preparation of such a beverage); frozen desserts such as ice cream, ice sherbet, and shaved ice; noodles such as buckwheat noodles, wheat noodles, bean-starch vermicelli, *gyoza* wraps (pot stickers), *su my* wraps (dim sum), Chinese noodles, and instant noodles; confectioneries such as chewing gum, candy, gummi candy, caramel, chocolate, tablet sweets, snacks, baked goods (e.g., biscuit), jelly, jam, and cream; fish-livestock processed foods such as minced and steamed fish, hamburger, ham, and sausage; dairy products such as processed milk, fermented milk, yogurt, butter, and cheese; fats and oils and fat and oil processed foods such as salad oil, tempura oil, margarine, mayonnaise, shortening, whipped cream, and dressing; seasonings such as sauce and baste; and soup, stew, curry, bread, jam, salad, daily dishes, and Japanese pickles. Examples of the same further include, but are not limited to, food products for nursing care and fluid diets for patients, in addition to foods or beverages for normal individuals.

The food or beverage of the present invention can also be mixed with ingredients other than the above collagen peptide composition. Examples of such ingredients include acidulants, saccharides, amino acids, various biologically active substances, vitamins, dietary fibers, polysaccharides, alcohols, and fats and oils.

Examples of an acidulant include organic acids such as citric acid, malic acid, tartaric acid, and acetic acid. Any types of saccharide may be used without particular limintation. Examples of saccharides include sucrose, malt sugar, fructose, glucose, invert sugar, powdered starch syrups, dextrin, and oligosaccharides. A sweetener with a high degree of sweetness such as aspartame, stevia, sucralose, or acesulfame potassium can also be used. Examples of amino acids include branched chain amino acids such as valine, leucine, and isoleucine, sulfur containing amino acids such as cysteine and methionine, and various other amino acids.

Examples of various biologically active substances include polyphenols such as isoflavone, anthocyanin, rutin, hesperidin, naringin, chlorogenic acid, gallic acid, ellagic acid, tannin, and catechin, saponin, lycopene, sesamin, ceramide, plant sterol, y-aminobutyric acid, coenzyme Q10, lactoferrin, DHA, and β carotin. Vitamins are not particularly limited and examples thereof include ascorbic acid (vitamin C), riboflavin, pantothenic acid, folic acid, B group vitamins, and other various vitamins such as vitamin A, vitamin D, vitamin E, vitamin K, and vitamin P.

Water soluble collagen, gelatin, and the like each having a molecular weight larger than that of a peptide can be combined. It is expected that functions and features that are unable to obtain by the use of the collagen peptide composition alone can be exerted through combination of a plurality of collagen ingredients. Moreover, particularly, cock's comb extract containing hyaluronic acid, a bovine, swine, or human placental extract, bovine or swine elastin and a hydrolysate thereof (obtainable by treatment with acid, alkali, and enzyme or the like) or a water soluble elastin derivative thereof, keratin and a hydrolysate thereof or a derivative thereof, a silk protein and a hydrolysate thereof or a derivative thereof, a hydrolysate of swine or bovine hemocyte protein (globin peptide), a decomposed product of bovine or swine hemoglobin (e.g., hemin, hematin, heme, protoheme, and heme iron), milk, casein and a hydrolysate thereof or a derivative thereof, a fat-free milk powder and a hydrolysate thereof or a derivative thereof, lactoferrin and a hydrolysate thereof, a hen egg ingredient, a decomposed product of fish meat, a nucleic acid-related substance (e.g., ribonucleic acid and deoxyribonucleic acid), or the like can also be added. Also, a plant peptide such as a soybean peptide can also be added.

Furthermore, an excipient, a binder, a diluent, a flavoring agent, a buffering agent, a thickener, a gelatinizing agent, a colorant, a stabilizer, an emulsifier, a dispersant, a suspending agent, an antiseptic, and the like can also be added.

The amount of the collagen peptide composition to be mixed with the food or beverage of the present invention may be any amount that allows the physiological and/or pharmacological effects to be exerted. In view of the general intake level of a target food or beverage, the amount can be set so that the intake level per day for an adult generally ranges from 100 mg to 10,000 mg, preferably ranges from 500 mg to 6,000 mg, and more preferably ranges from 1,000 mg to 3,000 mg. For example, in the case of a food in a solid form, the amount preferably ranges from 1% to 90% by weight and in the case of a liquid food such as a beverage, the amount preferably ranges from 0.1% to 20% by weight.

Typical examples of foods or beverages for mixing are listed specifically as follows, but the examples are not limited thereto.

Fruit juice beverage: collagen peptide composition (0.5 to 30 parts by weight), fruit juice (1 to 50 parts by weight), isomerized sugar syrup (5 to 20 parts by weight), acidulant (e.g., citric acid) (0.01 to 1.0 parts by weight), flavoring agent (0.1 to 1.0 parts by weight), and water (30 to 95 parts by weight).

Fruit jelly-jelly beverage: collagen peptide composition (0.5 to 20 parts by weight), fruit juice (1 to 40 parts by weight), granulated sugar (5 to 20 parts by weight), acidulant (e.g., citric acid) (0.01 to 1.0 parts by weight), gelatinizing agent (e.g., gelatin) (0.5 to 10.0 parts by weight), flavoring agent (0.1 to 1.0 parts by weight), and water (15 to 95 parts by weight).

Powdered food: collagen peptide composition (0.5 to 80 parts by weight), maltodextrin (5 to 20 parts by weight), thickener (e.g., gelatin) (0.1 to 5.0 parts by weight), emulsifier (e.g., sugar ester) (0.1 to 5.0 parts by weight), and sweetener (e.g., aspartame) (0.01 to 1 parts by weight).

Food in the form of tablet: Powders containing a combination of a collagen peptide composition (0.5 to 80 parts by weight), maltodextrin (5 to 20 parts by weight), a thickener (e.g., gelatin) (0.1 to 5.0 parts by weight), an emulsifier (e.g., sugar ester) (0.1 to 5.0 parts by weight), and a sweetener (e.g., aspartame) (0.01 to 1 parts by weight) are tabletted.

Various physiological and pharmacological effects are exerted through the oral ingestion of the food or beverage of the present invention, such as the curing of joint diseases (e.g., osteoarthritis and chronic rheumatism), the alleviation of osteoporosis, the prevention of arteriosclerosis and hypertension, the accelerated curing of wound sites, the curing of dermatological diseases (eczema, skin roughness, atopic dermatitis, pigment deposition, and bedsores), the improvement of moisture-retaining properties of skin, the improvement of skin aging (e.g., wrinkles, pigmented spots, dullness, sag, and keratinization), the prevention of hair aging (e.g., gray hair, hair loss, and thinning hair), and antiulcer effects.

### Examples

Hereafter, the present invention is described in greater detail with reference to the following examples, although the present invention is not limited to these examples.

### (Example 1) Preparation of the collagen peptide composition of the present invention (1)

Demineralized Tilapia scales were added to 8 times the amount of water to the amount of the scales. Sulfuric acid was added to the solution to adjust the pH to 2.0, the resultant was maintained for 3 hours, and thus acid treatment was carried out. Subsequently, the resultant was washed with water to remove excessive acid. Hot water was added to the scales after acid treatment. A gelatin solution was gradually collected while the solution was agitated at a temperature between 40°C and 90°C, followed by purification, sterilization, and drying to prepare fish scale gelatin. The thus prepared fish scale gelatin (1.0 kg) was dissolved in 2.0 kg of hot water at 75°C.

To the thus obtained gelatin solution, 20 g of purified papain (Trade name) (Mitsubishi-Kagaku Foods Corporation) (specific activity: 820 U/g) was added per kg of gelatin. The pH was adjusted to 5.5 and then an enzyme reaction was carried out at 60°C for 2 hours. After completion of the reaction, the solution was heated at 85°C or higher for 10 minutes so as to inactivate the enzyme. Pulverized activated carbon (20 g) was added, filtration was performed using filter cloth, microfiltration was carried out using a membrane filter, and then spray drying was carried out. Thus, a powdery collagen peptide composition 1 was obtained.

The average molecular weight of the thus obtained collagen peptide composition 1 was measured by carrying out gel filtration high performance liquid chromatography (GF-HPLC) under the following conditions. Data processing was carried out using Multistation GPC-8020 Software Ver 4.0 (TOSOH). The average molecular weight of the collagen peptide composition was calculated from the average retention time of the same using a calibration curve that had been separately prepared based on the retention time of a molecular weight marker for a molecular weight ranging from 307 to 17800 (glutathione: molecular weight of 307; oxytocin: molecular weight of 1007; insulin chain B: molecular weight of 3400; aprotinin: molecular weight of 6500; and myoglobin: molecular weight of 17800).

### (Analytical conditions)

Column: TSK-GEL 2500PW_{XL} (TOSOH, 300 × 7.8 mm)
Eluent: 45% acetonitrile (containing 0.1% trifluoroacetic acid)
Flow rate: 0.8 ml/min
Detection wavelength: 214 nm

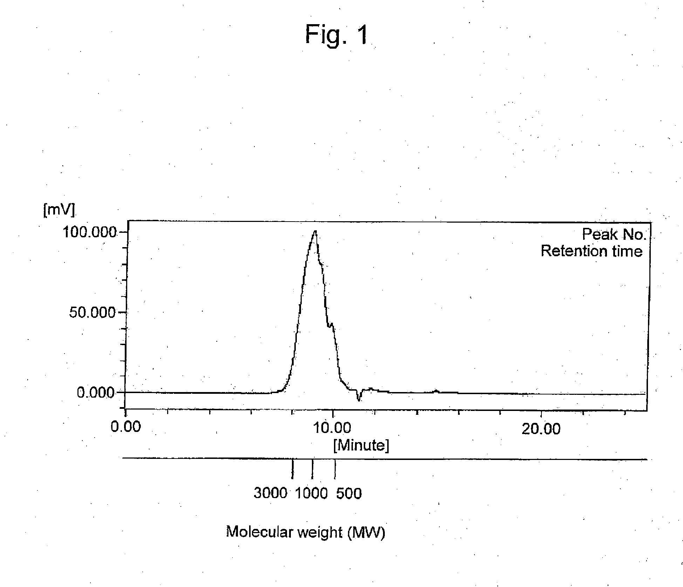

Fig. 1 shows the molecular weight distribution of the collagen peptide composition 1 of the present invention. The average molecular weight of the collagen peptide composition 1 was 1300.

### (Example 2) Preparation of the collagen peptide composition of the present invention (2)

To the gelatin solution obtained in Example 1, an enzyme mixture of purified papain (Trade name) (Mitsubishi-Kagaku Foods Corporation) and another protease [Neutrase (Trade name) (Novozymes Japan), protease P "Amano" 3G (Trade name) (Amano Enzyme Inc.), or protease N "Amano" G (Trade name) (Amano Enzyme Inc.)] was added. Enzyme reaction, enzyme inactivation, and purification treatment were carried out under the conditions shown in Table 1 below. Powdery collagen peptide compositions 2, 3, and 4 of the present invention were obtained.

**[Table 1]**

| Collagen peptide composition (sample name) | Name and amount of enzyme (per kg of gelatin) | Reaction pH, Reaction temperature, Reaction time | Inactivation conditions | Purification conditions |
|---|---|---|---|---|
| Collagen peptide composition 2 | Enzyme mixture of purified papain (20 g) and Neutrase (20 g) | pH 5.5, 60°C, 2 hours | 85°C or higher, 10 minutes | After addition of 2.0% by weight of pulverized activated carbon to gelatin, microfiltration |
| Collagen peptide composition 3 | Enzyme mixture of purified papain (20 g) and Protease P (20 g) | pH 5.5, 60°C, 2 hours | 85°C or higher, 10 minutes | After addition of 2.0% by weight of pulverized activated carbon to gelatin, microfiltration |
| Collagen peptide composition 4 | Enzyme mixture of purified papain (20 g) and Protease N (20 g) | pH 5.5, 60°C, 2 hours | 85°C or higher, 10 minutes | After addition of 2.0% by weight of pulverized activated carbon to gelatin, microfiltration |

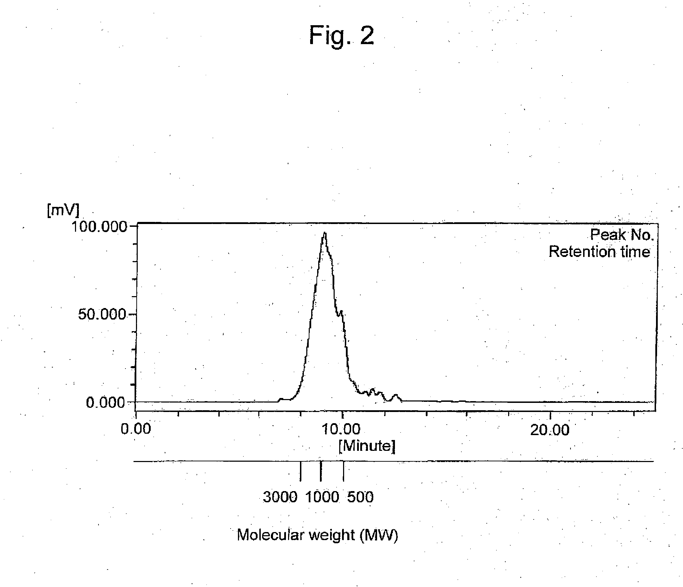

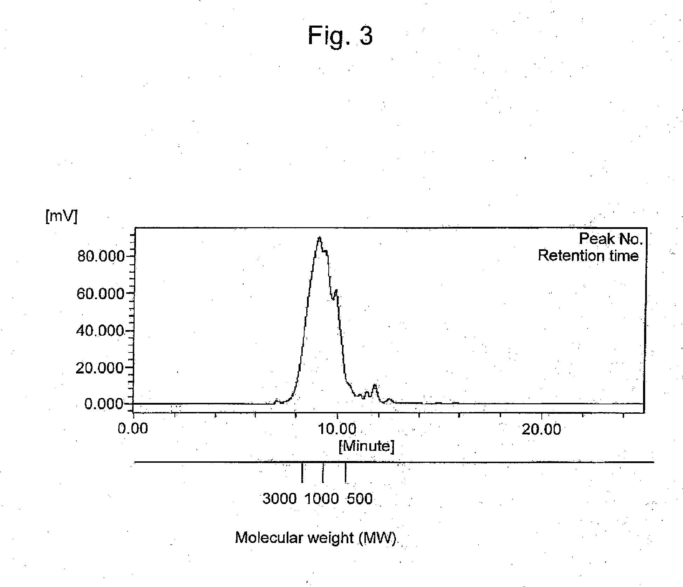

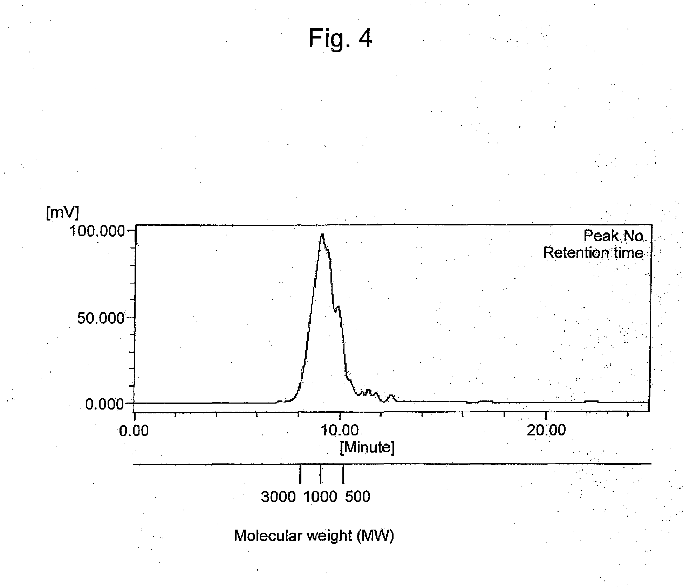

The molecular weight distributions of the collagen peptide compositions 2, 3, and 4 of the present invention are as shown in Fig. 2, Fig. 3, and Fig. 4. The average molecular weights of the thus obtained collagen peptide compositions 2, 3, and 4 were 1130, 1120, and 1080, respectively, as measured by a method similar to that used in Example 1.

### (Example 3) Preparation of a reference collagen peptide composition (3)

Pig skin gelatin (Rousselot, derived from pig skin) (1.0 kg) was dissolved in 2.0 kg of hot water at 75°C. Purified papain (Trade name) (Mitsubishi-Kagaku Foods Corporation) or an enzyme mixture of the above purified papain and Neutrase (Trade name) (Novozymes Japan) was added to the thus obtained gelatin solution. Enzyme reaction, enzyme inactivation, purification treatment were carried out under the conditions shown in Table 2 below. Thus, powdery collagen peptide compositions 5 and 6 were obtained.

**[Table 2]**

| Collagen peptide composition (sample name) | Name and amount of enzyme (per kg of gelatin) | Reaction pH, Reaction temperature, Reaction time | Inactivation conditions | Purification conditions |
|---|---|---|---|---|
| Collagen peptide composition 5 (reference) | Purified papain (20 g) | pH 5.5, 60°C, 13 hours | 85°C or higher, 10 minutes | After addition of 2.0% by weight of pulverized activated carbon to gelatin, micro filtration |
| Collagen peptide composition 6 (reference) | Enzyme mixture of purified papain (20 g) and Neutrase (20 g) | pH 5.5, 60°C, 13 hours | 85°C or higher, 10 minutes | After addition of 2.0% by weight of pulverized activated carbon to gelatin, microfiltration |

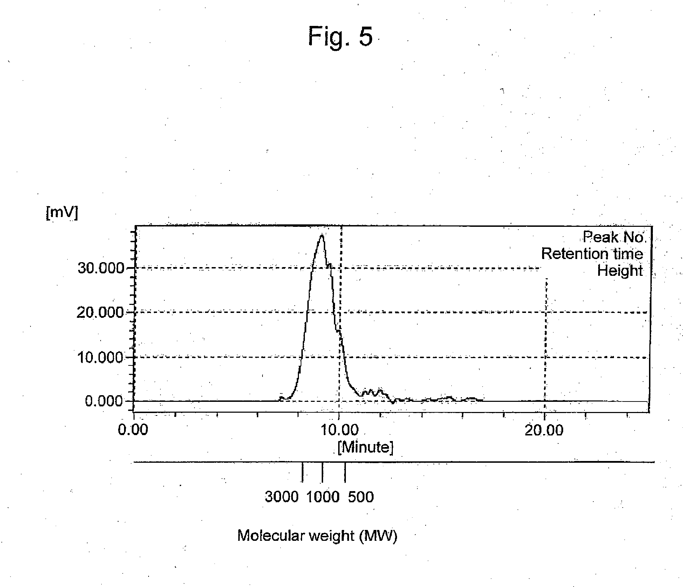

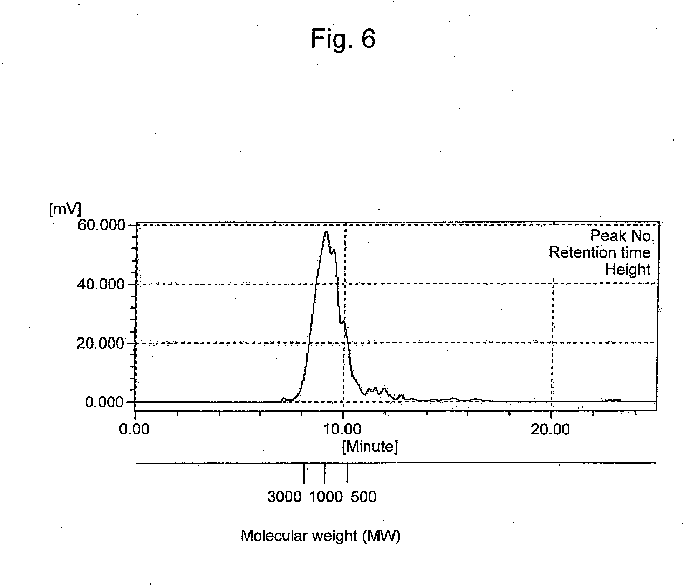

The molecular weight distributions of the collagen peptide compositions 5 and 6 are as shown in Fig. 5 and Fig. 6, respectively. The average molecular weights of the thus obtained collagen peptide compositions 5 and 6 measured by a method similar to that used in Example 1 were 1431 and 1313, respectively.

### (Test example 1) Test for ability to enter the blood (1)

The collagen peptide compositions 1-4 of the present invention and reference compositions 5 and 6 obtained in the above Examples were examined for ability to enter the blood. As products for comparison, commercial collagen peptide composition A (Ixos HDL-50F (Trade name), Nitta Gelatin Inc., fish scale-derived, average molecular weight: 5000) and collagen peptide composition E (collagen peptide described in Example 1 of WO2008/059927, fish scale-derived, and average molecular weight: 2000) were used.

The test for ability to enter the blood was conducted using 7-week-old male Hartley guinea pigs. The dose of a test sample was 3g/10mL/kg body weight. The sample was dissolved in distilled water and then the solution was orally administered. Guinea pigs were fasted from the evening of the day before the test. Blood was collected over time from guinea pigs under diethyl ether anaesthesia via the jugular vein before administration and at 0.5, 1, 2, and 6 hours after administration. Blood collection tubes in which blood had been collected were turned upside down several times for mixing, allowed to stand for approximately 15 minutes in ice, and then subjected to centrifugation (3000 rpm, 15 min, 4°C), so that blood plasma was obtained. Ethanol (900 µL) was added to 300 µL of blood plasma, the mixture was stirred using a Vortex for 15 seconds, and then the mixture was subjected to centrifugation (12,000 rpm, 10 min, 4°C), so that supernatants were obtained. The blood plasma collected from each guinea pig to which the test sample had been administered was cryopreserved at -80°C until it was used for analysis.

The levels of hydroxyproline-containing peptides in blood plasma are defined as the difference between the total hydroxyproline levels in blood plasma and the free hydroxyproline levels in blood plasma. The total hydroxyproline levels were measured according to the method of Sato et al., (Sato K. et al., J. Agric. Food Chem. 1992, 40, 806-810) by carrying out hydrolysis of blood plasma samples with 6N hydrochloric acid, carrying out treatment with phenylisothiocyanate (PITC) so as to generate PITC derivatives, and then carrying out HPLC under the following conditions. Also, the free hydroxyproline levels in blood plasma were measured for each deproteinated blood plasma sample in a manner similar to that employed for total hydroxyproline levels.

### (Analytical conditions)

Column: TSK80TsQA (TOSOH, 250 × 2.0 mm)
Eluent: (solution A) 50 mM sodium acetate buffer (pH 6) (solution B) acetonitrile
Elution conditions: solution B 5-10% (0-8 min), solution B 70% (8-11 min), solution B 5% (11 min)
Flow rate: 0.18 mL/min
Detection wavelength: 254 nm

Table 3 below shows the mean value ± S.E. of AUC₀₋₇ values (AUC: area under the curve of blood concentration of hydroxyproline-containing peptide-time) (hr·nmol/ml), as calculated for each blood plasma sample.

**[Table 3]**

| Blood hydroxyproline-containing peptide levels after the oral administration of collagen peptide compositions | |
|---|---|
| Collagen peptide composition | Hydroxyproline-containing peptide level (hr·nmol/ml: mean value ± S.E.) |
| Collagen peptide composition A (for comparison) | 218.7 ± 26.2 |
| Collagen peptide composition E (for comparison) | 224.0 ± 37.3 |
| Collagen peptide composition 1 | 41 5.7 ± 37. *1,*2 |
| Collagen peptide composition 2 | 406.4 ± 28.8 *1, *2 |
| Collagen peptide composition 3 | 376.6 ± 36.4 *1, *2 |
| Collagen peptide composition 4 | 367.6 ± 24.4 * 1, *2 |
| Collagen peptide composition 5 | 5 365.9 ± 33.9 *1, *2 |
| Collagen peptide composition 6 | 388.4 ± 30.5 *1, *2 |

| | |
|---|---|
| *1 : Intergroup study was carried out for collagen peptide composition A using Student's t-test. Results for which significant differences (p < 0.05) were found are indicated with *1. *2: Intergroup study was carried out for collagen peptide composition E using Student's t-test. Results for which significant differences (p < 0.05) were found are indicated with *2. | |

As shown in Table 3, when AUCs were compared, the ability to enter the blood of the collagen peptide compositions of the present invention had significantly increased to, 1.7 to 1.9 times that of the commercially available collagen peptide composition A and 1.6 to 1.85 times that of collagen peptide composition E.

### (Test example 2) Test for ability to enter the blood (2)

Collagen peptide compositions 1 and 2 of the present invention obtained in the above Examples were examined for the ability to enter the blood. For comparison, commercially available collagen peptide composition B (HACP (Trade name), (JELLICE Co., Ltd., pig skin-derived, and average molecular weight: 1500) was used.

The test was conducted based on the report of Iwai et al., (Agric. Food Chem., 2005, Vol. 53, No. 16, pp. 6531-6536). The test conducted herein was a crossover test such that a wash-out period of 6 or more days was set for 6 human volunteers, and each subject underwent a single instance of ingestion of the above 3 types of collagen peptide composition as test samples. Specifically, each subject was fasted for 12 hours, blood was collected before ingestion, and then the subject ingested each test sample. The intake level of each test sample was 5 g/subject. At 0, 0.5, 1, 2, 4, and 7 hours after ingestion, 5 mL of blood was collected and then the level of hydroxyproline-containing peptide in blood plasma was found by a method similar to that used in Test example 1. Table 4 below shows the AUC₀₋₇ values (AUC: area under the curve of blood concentration of hydroxyproline-containing peptide-time) (hr.nmol/ml: mean value ± S.E.), as calculated for each blood plasma sample.

**[Table 4]**

| Blood hydroxyproline-containing peptide levels after the oral administration of collagen peptide compositions | |
|---|---|
| Collagen peptide composition | Hydroxyproline-containing peptide level (hr·nmol/ml: mean value ± S.E.) |
| Collagen peptide composition B (for comparison) | 48.8 ± 2.5 |
| Collagen peptide composition 1 | 71.4 ± 8.5* |
| Collagen peptide composition 2 | 74.7 ± 3.7* |

| | |
|---|---|
| *: Intergroup study was carried out for collagen peptide composition B using Student's t-test. Results for which significant differences (p < 0.05) were found are indicated with *. | |

As shown in Table 4, when AUCs were compared, the ability to enter the blood of the collagen peptide compositions 1 and 2 of the present invention had significantly increased to 1.46 to 1.53 times that of the commercially available collagen peptide composition B.

### (Test example 3) Determination of N terminal amino acids of collagen peptide compositions

Collagen peptide compositions 1 to 6 of the present invention and the following collagen peptide compositions A to F as products for comparison were examined for amino acid sequences and the amounts of specific amino acids.
Collagen peptide composition A: Ixos HDL-50F (Trade name) (Nitta Gelatin Inc., fish scale-derived, and average molecular weight: 5000)
Collagen peptide composition B: HACP (Trade name) (JELLICE Co., Ltd., pig skin-derived, and average molecular weight: 1500)
Collagen peptide composition C: Nippi Peptide FCP (Trade name) Nippi, inc., fishskin-derived, and average molecular weight: 5000)
Collagen peptide composition D: Nippi Peptide FCP-A (Trade name) (Nippi, inc., fishskin-derived, and average molecular weight: 5000)
Collagen peptide composition E: Example 1 of WO2008/059927 (fish scale-derived, and average molecular weight: 2000),
Collagen peptide composition F: Marine collagen MS5 (Trade name) RABJ CO., LTD, fish scale-derived, and average molecular weight: 8000)

The amino acid sequences of peptides contained in each collagen peptide were determined by dissolving each peptide in water, adding the solution to a PVDF membrane dropwise, and then carrying out analysis using a protein sequencer (PPSQ) (Shimadzu Corporation) that is an amino acid sequence analyzer used for carrying out the Edman method in an automated manner. Table 5 below shows the ratio of hydroxyproline to total of amino acid residues at the second position from the N terminus of the peptides in each composition and the ratio of glycine to total of amino acid residues at the third position from the N terminus of the same.

**[Table 5]**

| Collagen peptide composition | The ratio of hydroxyproline to total of amino acid residues at the second position from the N terminus (mol%) | The ratio of glycine to total of amino acid residues at the third position from the N terminus (mol%) |
|---|---|---|
| Collagen peptide composition 1 | 7.4 | 41.9 |
| Collagen peptide composition 2 | 6.9 | 43.9 |
| Collagen peptide composition 3 | 6.5 | 35.2 |
| Collagen peptide composition 4 | 8.7 | 35.8 |
| Collagen peptide composition 5 | 6.8 | 42.2 |
| Collagen peptide composition 6 | 3.8 | 42.2 |
| Collagen peptide composition A | 0.7 | 9.8 |
| Collagen peptide composition B | 0.5 | 1.3 |
| Collagen peptide composition C | 1.6 | 14.6 |
| Collagen peptide composition D | 1.0 | 10.5 |
| Collagen peptide composition E | 0.8 | 9.8 |
| Collagen peptide composition F | 1.8 | 19.0 |

As shown in Table 5, whereas the ratio of hydroxyproline to total of amino acid residues at the second position from the N terminus in each of the collagen compositions 1 to 6 of the present invention ranges from 3.8 mol% to 8.7 mol%, the same in each of the collagen peptide compositions A to F as products for comparison ranges from 0.5 mol% to 1.8 mol%. Also, whereas the ratio of glycine to total of amino acid residues at the third position from the N terminus in each of the collagen compositions 1 to 6 of the present invention ranges from 35.2 mol% to 43.9 mol%, the same in each of the collagen peptide compositions A to F as products for comparison ranges from 1.3 mol% to 19.0 mol%.

### Industrial Applicability

The present invention can be used in the fields of production of foods or beverages such as functional foods and supplements.

## Claims

1. A collagen peptide composition obtainable by digesting gelatinized collagen or gelatin derived from fish scale with protease for 0.5 to 4 hours at 30 to 80°C;
wherein the gelatinized collagen or gelatin is prepared by acid treatment of collagen raw materials; and wherein
(a) the ratio of hydroxyproline to total of amino acid residues at the second position from the N terminus of the peptides in the composition is 2 mol% or more and 20 mol% or less and the ratio of glycine to total of amino acid residues at the third position from the N terminus of the same is 20 mol% or more and 50 mol% or less; and
(b) the average molecular weight is 1000 or more and 1500 or less;
(c) the protease is papain alone or an enzyme mixture of papain and one, two or more types of neutral protease from the genus Aspergillus or the genus Bacillus, or neutral protease from the genus Rhizopus; and
(d) the papain has specific activity ranging from 400 U/g to 5000 U/g.

2. The collagen peptide composition according to claim 1, wherein the total activity of the papain in 100 g of collagen ranges from 400 U to 5000 U.

3. A food or beverage, containing the collagen peptide composition according to claims 1 or 2.

4. A method for producing a collagen peptide composition, comprising digesting gelatinized collagen or gelatin derived from fish scale with protease for 0.5 to 4 hours at 30 to 80°C; wherein the gelatinized collagen or gelatin is prepared by acid treatment of collagen raw materials; and wherein
(a) the ratio of hydroxyproline at the second position from the N terminus to total of amino acid residues at the second position from the N terminus of the peptides in the composition is from 2 mol% to 20 mol% and the ratio of glycine at the third position from the N terminus to total of amino acid residues at the third position from the N terminus of the same is from 20 mol% to 50 mol%;
(b) the average molecular weight of the composition is from 1000 to 1500;
(c) the protease is papain alone or an enzyme mixture of papain and one, two or more types of neutral protease from the genus Aspergillus or the genus Bacillus, or neutral protease from the genus Rhizopus; and
(d) the papain has specific activity ranging from 400 U/g to 5000 U/g.

5. The method according to claim 4, wherein the total activity of the papain in 100 g of collagen ranges from 400 U to 5000 U.

6. Use of the collagen peptide composition according to claims 1 or 2 in the production of food or beverage.

## Patentansprüche

1. Kollagenpeptid-Zusammensetzung, die durch Verdauen von gelatiniertem Kollagen oder Gelatine, das/die aus Fischschuppen stammt, mit Protease für 0,5 bis 4 Stunden bei 30 bis 80°C erhalten werden kann;
worin das gelatinierte Kollagen oder die Gelatine durch Säurebehandlung von Kollagen-Rohmaterial hergestellt wird; und worin
(a) das Verhältnis von Hydroxyprolin zur Gesamtheit der Aminosäurereste an der zweiten Position von dem N-Terminus der Peptide in der Zusammensetzung 2 mol-% oder mehr und 20 mol-% oder weniger beträgt und das Verhältnis von Glycin zur Gesamtheit der Aminosäurereste an der dritten Position von dem N-Terminus der gleichen 20 mol-% oder mehr und 50 mol-% oder weniger beträgt; und
(b) das durchschnittliche Molekulargewicht 1000 oder mehr und 1500 oder weniger beträgt;
(c) die Protease Folgendes ist: nur Papain oder eine Enzymmischung aus Papain und einem, zwei oder mehr Typen von neutraler Protease aus der Gattung Aspergillus oder der Gattung Bacillus oder von neutraler Protease aus der Gattung Rhizopus; und
(d) das Papain eine spezifische Aktivität im Bereich von 400 U/g bis 5000 U/g aufweist.

2. Kollagenpeptid-Zusammensetzung nach Anspruch 1, worin die gesamte Aktivität des Papains in 100 g Kollagen im Bereich von 400 U bis 5000 U liegt.

3. Lebensmittel oder Getränk, das die Kollagenpeptid-Zusammensetzung nach Anspruch 1 oder 2 enthält.

4. Verfahren zur Herstellung einer Kollagenpeptid-Zusammensetzung, das das Verdauen von gelatiniertem Kollagen oder Gelatine, das/die aus Fischschuppen stammt, mit Protease für 0,5 bis 4 Stunden bei 30 bis 80°C umfasst;
worin das gelatinierte Kollagen oder die Gelatine durch Säurebehandlung von Kollagen-Rohmaterial hergestellt wird; und worin
(a) das Verhältnis von Hydroxyprolin an der zweiten Position von dem N-Terminus zur Gesamtheit der Aminosäurereste an der zweiten Position von dem N-Terminus der Peptide in der Zusammensetzung von 2 mol-% bis 20 mol-% beträgt und das Verhältnis von Glycin an der dritten Position von dem N-Terminus zur Gesamtheit der Aminosäurereste an der dritten Position von dem N-Terminus der gleichen von 20 mol-% bis 50 mol-% beträgt;
(b) das durchschnittliche Molekulargewicht der Zusammensetzung von 1000 bis 1500 beträgt;
(c) die Protease Folgendes ist: nur Papain oder eine Enzymmischung aus Papain und einem, zwei oder mehr Typen von neutraler Protease aus der Gattung Aspergillus oder der Gattung Bacillus oder von neutraler Protease aus der Gattung Rhizopus; und
(d) das Papain eine spezifische Aktivität im Bereich von 400 U/g bis 5000 U/g aufweist.

5. Verfahren nach Anspruch 4, worin die gesamte Aktivität des Papains in 100 g Kollagen im Bereich von 400 U bis 5000 U liegt.

6. Verwendung der Kollagenpeptid-Zusammensetzung nach den Ansprüchen 1 oder 2 bei der Herstellung von Lebensmitteln oder Getränken.

## Revendications

1. Composition peptidique de collagène pouvant être obtenue par digestion de collagène gélatinisé ou de gélatine dérivée d'écaille de poisson avec de la protéase pendant 0,5 à 4 heures à 30 à 80° C ;
dans laquelle le collagène gélatinisé ou la gélatine est préparé(e) par acidification des matières brutes du collagène ; et dans laquelle
(a) le rapport d'hydroxyproline sur le total de résidus d'acides aminés au niveau de la deuxième position depuis l'extrémité N des peptides dans la composition est au minimum de 2 mol % et au maximum de 20 mol % et le rapport de glycine sur le total de résidus d'acides aminés au niveau de la troisième position depuis l'extrémité N de ceux-ci est au minimum de 20 mol % et au maximum de 50 mol % ; et
(b) la masse moléculaire moyenne est au minimum de 1000 et au maximum de 1500 ;
(c) la protéase est de la papaïne seule ou un mélange enzymatique de papaïne et d'un, de deux types ou plus de protéase neutre provenant du genre Aspergillus ou du genre Bacillus, ou de protéase neutre provenant du genre Rhizopus ; et
(d) la papaïne a une activité spécifique variant de 400 U/g à 5 000 U/g.

2. Composition peptidique de collagène selon la revendication 1, dans laquelle l'activité totale de la papaïne dans 100 g de collagène varie de 400 U à 5 000 U.

3. Aliment ou boisson, contenant la composition peptidique de collagène selon la revendication 1 ou la revendication 2.

4. Procédé de production d'une composition peptidique de collagène, comprenant de digérer du collagène gélatinisé ou de la gélatine dérivée d'écaille de poisson avec de la protéase pendant 0,5 à 4 heures à 30 à 80° C ; dans lequel le collagène gélatinisé ou la gélatine est préparé(e) par acidification des matières brutes du collagène ; et dans lequel
(a) le rapport d'hydroxyproline au niveau de la deuxième position depuis l'extrémité N sur le total de résidus d'acides aminés au niveau de la deuxième position depuis l'extrémité N des peptides dans la composition est de 2 mol % à 20 mol % et le rapport de la glycine au niveau de la troisième position depuis l'extrémité N sur le total de résidus d'acides aminés au niveau de la troisième position depuis l'extrémité N de ceux-ci est de 20 mol % à 50 mol % ;
(b) la masse moléculaire moyenne de la composition est de 1000 à 1500 ;
(c) la protéase est de la papaïne seule ou un mélange enzymatique de papaïne et d'un, de deux types ou plus de protéase neutre provenant du genre Aspergillus ou du genre Bacillus, ou de protéase neutre provenant du genre Rhizopus ; et
(d) la papaïne a une activité spécifique variant de 400 U/g à 5 000 U/g.

5. Procédé selon la revendication 4, dans lequel l'activité totale de la papaïne dans 100 g de collagène varie de 400 U à 5 000 U.

6. Utilisation de la composition peptidique de collagène selon la revendication 1 ou la revendication 2 dans la production d'aliment ou de boisson.
